# EUROPÄISCHE PATENTSCHRIFT

(11) **EP 1 470 786 B1**
(45) Veröffentlichungstag und Bekanntmachung des Hinweises auf die Patenterteilung: **05.10.2005**
(21) Anmeldenummer: 03009438.7
(22) Anmeldetag: 25.04.2003
(51) Int. Cl.: A61B 17/15

(54) **Vorrichtung zur Vorbereitung einer Femurkondyle**
Device for preparation of a femoral condyle
Dispositif pour la préparation d'un condyle d'un fémur

(43) Veröffentlichungstag der Anmeldung: 27.10.2004
(73) Patentinhaber: Zimmer GmbH, 8404 Winterthur (CH)
(72) Erfinder: Faoro, Francisco, 8002 Zürich (CH); Overes, Tom, 8400 Winterthur (CH)
(74) Vertreter: Manitz, Finsterwald & Partner GbR

(56) Entgegenhaltungen:
- EP-A- 0 709 061
- WO-A-01/66021
- WO-A-01/85038

## Beschreibung

Die Erfindung betrifft eine Vorrichtung zur Vorbereitung einer Femurkondyle beim Einsetzen von monokondylären Knieimplantaten.

Beim Einsetzen von monokondylären, d.h. einseitigen, Knieprothesen müssen die Kondylen der Tibia und des Femur vorbereitet werden, um Anlageflächen an den Knochen zu schaffen, die eine definierte Lage des Tibia- und Femurimplantats der Knieprothese gewährleisten.

Die Anlageflächen werden durch Wegschneiden von Knochenmaterial an den Kondylen erzeugt. Dabei ist man bestrebt, so wenig Knochenmaterial wie möglich zu entfernen. Ferner muss darauf geachtet werden, dass die Schnittflächen an der Tibia und am Femur richtig relativ zueinander orientiert sind, damit das Tibia- und Femurimplantat in einer der natürlichen Bewegung des restlichen, gesunden Teils des Kniegelenks entsprechenden Weise zusammenwirken kann.

Wenn im Verlauf einer Knieoperation zuerst die Tibia vorbereitet und hierbei ein Tibiaplateau erzeugt wird, auf das später ein Tibiaimplantat aufgesetzt wird, dann kommt es bei der Vorbereitung der entsprechenden Femurkondyle darauf an, dass die an der Femurkondyle zu erzeugenden Schnittflächen korrekt relativ zu den am Tibiaplateau erzeugten Schnittflächen ausgerichtet sind.

Ein Verfahren Arthroplastik an Kniegelenken sowie ein hierfür verwendetes Instrumentarium einschließlich eines Schnittblocks sind aus WO 01/66021 A1 bekannt.

Aufgabe der Erfindung ist es, eine Vorrichtung der eingangs genannten Art zu schaffen, mit der auf möglichst einfache und zuverlässige Weise ein Femurimplantat mit möglichst hoher Genauigkeit in der richtigen Lage relativ zur Tibia am Femur implantiert werden kann.

Die Lösung dieser Aufgabe erfolgt durch die Merkmale des Anspruchs 1 und insbesondere dadurch, dass wenigstens eine kombinierte Schnitt- und Bohrlehre vorgesehen ist, die bei in Flexion befindlichem Knie in einem von der Dicke eines einzusetzenden Tibiaimplantats abhängigen Sollabstand von einem Tibiaplateau an der Femurkondyle fixierbar ist, deren der Femurkondyle zugewandte konkave Seite entsprechend einem einzusetzenden Femurimplantat gekrümmt ist, die in einem Bohrlehrenabschnitt wenigstens einen Fixierdurchgang für einen Bohrer und für ein Fixierelement aufweist, wobei der Fixierdurchgang bezüglich der gekrümmten Seite entsprechend dem einzusetzenden Femurimplantat positioniert und orientiert ist, die in einem Schnittlehrenabschnitt wenigstens einen Schlitz für ein Schneidewerkzeug aufweist, durch den eine Schnittebene für einen Kondylenschnitt definiert ist, und die einen Kopplungsabschnitt für eine Ausrichthilfe aufweist, mittels welcher die im Sollabstand vom Tibiaplateau befindliche Schnitt- und Bohrlehre relativ zur Femurkondyle verstellbar ist.

Mit der erfindungsgemäßen kombinierten Schnitt- und Bohrlehre wird ein Multifunktionsinstrument geschaffen, mit dem bei der Vorbereitung der Femurkondyle das an dieser zu implantierende Femurimplantat insofern simuliert wird, als die konkave Seite der Lehre entsprechend dem Femurimplantat gekrümmt ist und der Fixierdurchgang der Lehre hinsichtlich Lage und Orientierung relativ zu der gekrümmten Seite ebenfalls dem Femurimplantat entspricht. Die mittels der erfindungsgemäßen Schnitt- und Bohrlehre auszuführenden Schnitte und Bohrungen sind folglich optimal auf das eigentliche Femurimplantat abgestimmt, und zwar auch im Hinblick auf die Position und die Orientierung relativ zu dem Tibiaplateau, da erfindungsgemäß die Schnitte und Bohrungen bei dem richtigen Sollabstand von dem Tibiaplateau ausgeführt werden können und darüber hinaus durch den für die Ausrichthilfe vorgesehenen Kopplungsabschnitt eine zusätzliche Verstellmöglichkeit relativ zur Femurkondyle besteht. Diese zusätzliche Verstellmöglichkeit ermöglicht eine besonders exakte Ausrichtung der kombinierten Schnitt- und Bohrlehre in Abhängigkeit von den jeweiligen anatomischen Gegebenheiten.

Von dem aus WO 01/66021 A1 bekannten Schnittblock unberscheidet sich die erfindungsgemäße Vorrichtung dadurch, dass die Schnitt- und Bohrlehre wie das einzusetzende Femurimplantat gekrummt und an der Femurkondyle fixierbar ist.

Vorteilhafte Ausführungsformen der Erfindung sind in den Unteransprüchen, der Beschreibung sowie der Zeichnung angegeben.

Als Kopplungsabschnitt der Schnitt- und Bohrlehre kann wenigstens ein im Bohrlehrenabschnitt ausgebildeter Kopplungsdurchgang vorgesehen sein, der im Wesentlichen senkrecht zu dem Fixierdurchgang verläuft. Hierdurch wird eine seitliche Ankoppelung der Ausrichthilfe ermöglicht.

In einem besonders bevorzugten Ausführungsbeispiel ist die Schnitt- und Bohrlehre mit einer Aufspreizeinrichtung koppelbar, mittels welcher ein Sollabstand zwischen der Femurkondyle und einem gegenüberliegenden Tibiaplateau einstellbar ist.

Mittels einer derartigen Aufspreizeinrichtung können durch Aufspreizen des Knies, d.h. durch Vergrößern des Abstandes zwischen Femur und Tibia, während der Knieoperation das Femur und die Tibia in ihre natürliche Relativlage gebracht werden, wobei diese natürliche Relativlage anhand der natürlichen Spannung der Bänder vom Operateur ermittelt werden kann. Vorzugsweise ist die Aufspreizeinrichtung so ausgebildet, dass die erfindungsgemäße Schnitt- und Bohrlehre derart mit der Aufspreizeinrichtung gekoppelt werden kann, dass die Schnitt- und Bohrlehre relativ zur Aufspreizeinrichtung in der Höhe verstellbar ist, und zwar bei mittels der Aufspreizeinrichtung aufgespreiztem Knie, d.h. bei eingestelltem Sollabstand zwischen Tibia und Femur.

Die Aufspreizeinrichtung selbst ist nicht Gegenstand der vorliegenden Erfindung, so dass auf Details hierzu im Folgenden nicht näher eingegangen wird.

In einer weiteren Ausführungsform der Erfindung ist der Schnittlehrenabschnitt der Schnitt- und Bohrlehre gleichzeitig als mit der Aufspreizeinrichtung koppelbarer Gleitsteckschuh ausgebildet, über den die Schnitt- und Bohrlehre an der Aufspreizeinrichtung zwangsgeführt verstellbar ist.

Ferner kann vorgesehen sein, dass die Schnitt- und Bohrlehre zusätzlich zu dem Fixierdurchgang mit einem im Bohrlehrenabschnitt ausgebildeten Positionierdurchgang versehen ist, über den die Schnitt- und Bohrlehre vor der über den Fixierdurchgang erfolgenden Fixierung an der Femurkondyle mittels eines Positionierstiftes relativ zur Femurkondyle positionierbar ist.

In einem weiteren Ausführungsbeispiel der Erfindung ist eine zusätzliche Schnittlehre vorgesehen, mit welcher die Schnitt- und Bohrlehre koppelbar ist und mit der bei in Flexion befindlichem Knie ein weiterer Kondylenschnitt festgelegt werden kann, der gekrümmt zwischen zwei zuvor an der Femurkondyle hergestellten ebenen Schnittflächen verläuft, von denen die eine Schnittfläche mittels des Schnittlehrenabschnitts der kombinierten Schnitt- und Bohrlehre und die andere Schnittfläche bei in Extension befindlichem Knie ausgebildet wurde. Die beiden ebenen Schnittflächen laufen vorzugsweise zumindest im Wesentlichen senkrecht zueinander.

Bevorzugt ist ferner vorgesehen, dass mittels der zusätzlichen Schnittlehre der Verlauf der gekrümmten Seite der kombinierten Schnitt- und Bohrlehre zumindest bereichsweise auf der Femurkondyle abbildbar ist. Da die Schnitt- und Bohrlehre mit ihrer gekrümmten Seite entsprechend dem eigentlichen Femurimplantat geformt ist, kann durch das Zusammenwirken der Schnitt- und Bohrlehre mit der zusätzlichen Schnittlehre die Femurkondyle exakt an die entsprechende Seite des Femurimplantats angepasst werden.

Die zusätzliche Schnittlehre kann eine Kurvenscheibe mit einer konvexen Führungsfläche umfassen, entlang welcher ein Schneidewerkzeug führbar ist und deren Verlauf der gekrümmten Seite der kombinierten Schnitt-und Bohrlehre entspricht.

Des Weiteren kann erfindungsgemäß vorgesehen sein, dass die zusätzliche Schnittlehre im mit der an der Femurkondyle fixierten Schnitt- und Bohrlehre gekoppelten Zustand derart bezüglich der Schnitt- und Bohrlehre orientiert ist, dass die Führungsfläche der zusätzlichen Schnittlehre und die gekrümmte Seite der Schnitt- und Bohrlehre ausschließlich translatorisch gegeneinander versetzt sind.

Die zusätzliche Schnittlehre kann des Weiteren derart ausgebildet sein, dass sie im mit der an der Femurkondyle fixierten Schnitt- und Bohrlehre gekoppelten Zustand ebenfalls an der Femurkondyle fixierbar ist.

Des Weiteren wird erfindungsgemäß vorgeschlagen, dass die zusätzliche Schnittlehre mit wenigstens einem und vorzugsweise einer Mehrzahl von Fixierdurchgängen versehen ist, die sich im mit der kombinierten Schnitt- und Bohrlehre gekoppelten Zustand im Wesentlichen senkrecht zu dem Fixierdurchgang der kombinierten Schnitt- und Bohrlehre erstrecken.

Gemäß einem weiteren bevorzugten Ausführungsbeispiel der Erfindung ist die Führungsfläche bei an der Femurkondyle fixierter zusätzlicher Schnittlehre relativ zu der Femurkondyle verstellbar. Diese Verstellbarkeit der zusätzlichen Schnittlehre ermöglicht es, im Bedarfsfall eine letzte Nachjustage der Führungsfläche vorzunehmen, bevor der weitere Kondylenschnitt ausgeführt wird.

Die zusätzliche Schnittlehre kann einen an der Femurkondyle fixierbaren Basisabschnitt umfassen, an dem die Kurvenscheibe bei an der Femurkondyle fixiertem Basisabschnitt zur Ausrichtung der an der Kurvenscheibe ausgebildeten Führungsfläche mit einem Kondylenschnitt verstellbar angebracht ist, der zuvor bei in Extension befindlichem Knie in einem von der Dicke eines einzusetzenden Tibiaimplantats abhängigen Sollabstand vom Tibiaplateau ausgeführt wurde.

Dabei kann die Kurvenscheibe derart relativ zu dem an der Femurkondyle fixierten Basisabschnitt verstellbar sein, dass der Scheitelpunkt der an der Kurvenscheibe ausgebildeten Führungsfläche in der durch den Kondylenschnitt definierten Ebene gelegen ist.

Ferner wird erfindungsgemäß vorgeschlagen, dass der für die Ausrichthilfe vorgesehene Kopplungsabschnitt der kombinierten Schnitt- und Bohrlehre gleichzeitig zur Koppelung mit der zusätzlichen Schnittlehre ausgebildet ist.

Gemäß einer weiteren Ausführungsform der Erfindung ist vorgesehen, dass zur Koppelung der zusätzlichen Schnittlehre mit der kombinierten Schnitt- und Bohrlehre eine separate Kopplungseinrichtung vorgesehen ist, an der die zusätzliche Schnittlehre fixierbar und mit der die zusätzliche Schnittlehre im fixierten Zustand relativ zu der kombinierter Schnitt- und Bohrlehre verstellbar, insbesondere linear verschiebbar, ist.

Die Kopplungseinrichtung kann eine Spannvorrichtung umfassen, mittels welcher die zusätzliche Schnittlehre an der Kopplungseinrichtung fixerend eingespannt werden kann.

Des Weiteren ist die Kopplungseinrichtung vorzugsweise derart ausgebildet, dass sie abgenommen werden kann, wenn sowohl die kombinierte Schnitt- und Bohrlehre als auch die zusätzlicher Schnittlehre an der Femurkondyle fixiert sind.

Vorzugsweise sind sowohl die kombinierte Schnitt- und Bohrlehre als auch die zusätzliche Schnittlehre jeweils in verschiedenen Größen vorgesehen, die unterschiedlich großen Femurimplantaten entsprechen. Folglich wird in Abhängigkeit von der gemäß der Operationsplanung ausgewählten Größe des einzusetzenden Femurimplantats aus dem Satz unterschiedlich großer Schnitt- und Bohrlehren sowie aus dem Satz unterschiedlich großer zusätzlicher Schnittlehren jeweils das größenmäßig auf das einzusetzende Femurimplantat abgestimmte Exemplar ausgewählt, wodurch eine exakte Vorbereitung der Femurkondyle zum Einsetzen des Femurimplantats sichergestellt ist.

Die Erfindung wird im Folgenden beispielhaft unter Bezugnahme auf die Zeichnung beschrieben. Es zeigen:
- Fig. 1 bis 7 u. 11: verschiedene Phasen eines Teils einer Knieoperation, bei dem mittels einer erfindungsgemäßen Vorrichtung eine Femurkondyle zum Einsetzen eines Femurimplantats vorbereitet wird,
- Fig. 8: verschiedene Ansichten einer kombinierten Schnitt- und Bohrlehre gemäß einer Ausführungsform der Erfindung,
- Fig. 9: verschiedene Ansichten einer separaten, mit der Schnitt- und Bohrlehre von Fig. 8 koppelbaren Kopp- lungseinrichtung gemäß einer Ausführungsform der Er- findung, und
- Fig. 10: eine mit der Kopplungseinrichtung von Fig. 9 koppelba- re zusätzliche Schnittlehre gemäß einer Ausführungs- form der Erfindung.

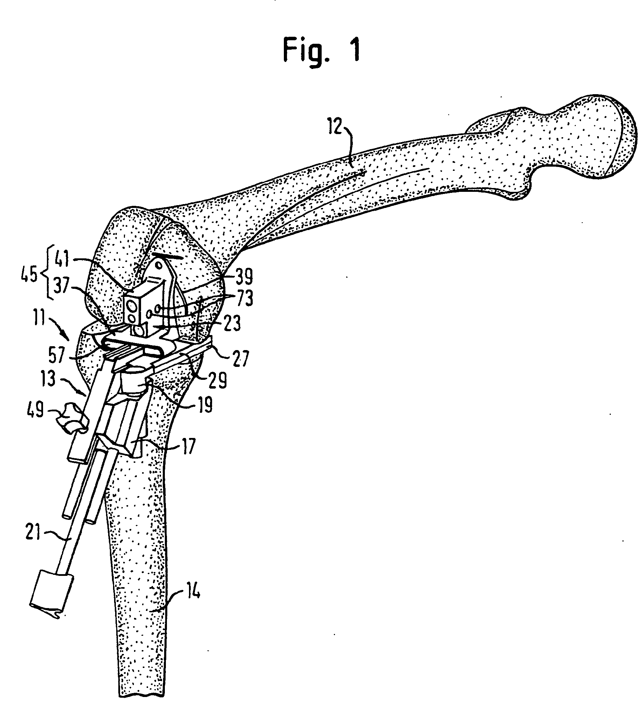

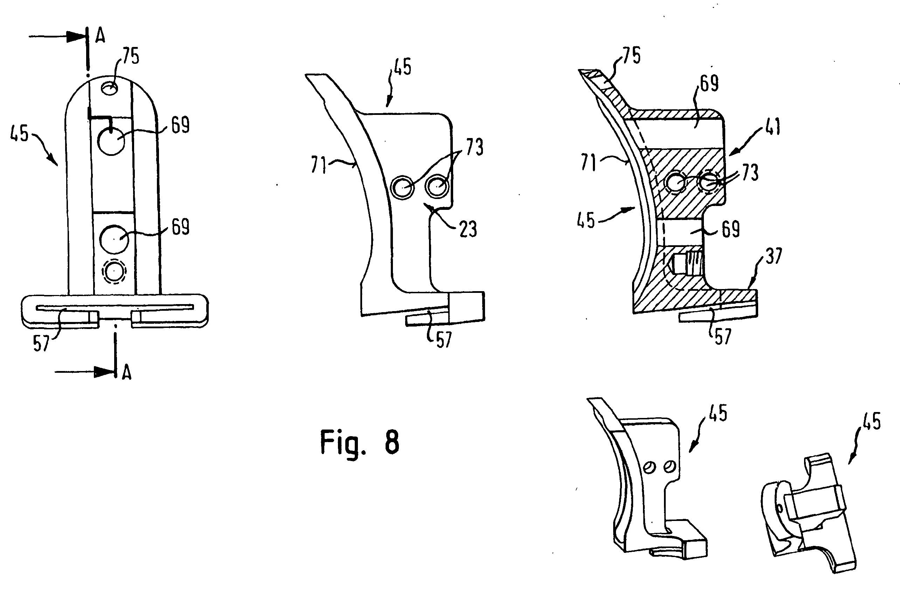

Bevor anhand der Fig. 8 bis 10 auf konstruktive Details der erfindungsgemäßen Vorrichtung eingegangen wird, soll zunächst anhand der Fig. 1 bis 7 und 11 derjenige Teil einer Knieoperation erläutert werden, bei dem die erfindungsgemäße Vorrichtung zum Einsatz kommt.

Bei der Operation, in der die erfindungsgemäße Vorrichtung gemäß dem in den Figuren gezeigten Ausführungsbeispiel zum Einsatz kommt, handelt es sich um eine unikompartimentale Operation, bei der entweder im lateralen oder im medialen Kompartiment die Tibiakondyle und die Femurkondyle zum Einsetzen eines Tibiaimplantats bzw. Femurimplantats vorbereitet wird.

Ausgangspunkt für denjenigen Teil der Operation, in dem die erfindungsgemäße Vorrichtung verwendet wird, ist hier ein durch zwei senkrecht zueinander verlaufende Knochenschnitte hergestelltes Tibiaplateau, auf das ein gemäß der Operationsplanung vorgesehenes Tibiaimplantat aufgesetzt werden kann, sobald mit Hilfe der erfindungsgemäßen Vorrichtung auch die gegenüberliegende Femurkondyle zur Fixierung des entsprechenden Femurimplantats vorbereitet ist.

Das erfindungsgemäße Instrumentarium umfasst gemäß dem im Folgenden beschriebenen Ausführungsbeispiel eine einstückig ausgebildete kombinierte Schnitt- und Bohrlehre 45, die einen Bohrlehrenabschnitt 41 und einen Schnittlehrenabschnitt 37 umfasst.

Im Anschluss an die Herstellung des Tibiaplateaus, die bei in Extension befindlichem Knie erfolgt, wird zunächst ebenfalls bei in Extension befindlichem Knie ein parallel zum Tibiaplateau verlaufender erster Kondylenschnitt 39 ausgeführt. Dies erfolgt mittels einer Aufspreizeinrichtung 11, die auch in Verbindung mit der erfindungsgemäßen kombinierten Schnitt- und Bohrlehre 45 verwendet wird (vgl. Fig. 1). Anstelle der erfindungsgemäßen Schnitt- und Bohrlehre 45 ist zur Ausführung des ersten Kondylenschnittes 39 bei in Extension befindlichem Knie ein anderer Funktionsaufsatz mit der Aufspreizeinrichtung 11 gekoppelt, der eine Schnittlehre umfasst, durch welche die Schnittebene für den ersten Kondylenschnitt 39 definiert ist.

Mit der Aufspreizeinrichtung 11 wird das Knie aufgespreizt, d.h. der Abstand zwischen Tibia 14 und Femur 12 vergrößert, bis Femur 12 und Tibia 14 in ihre natürliche Relativlage gebracht sind, die der Operateur anhand der natürlichen Spannung der Bänder ermitteln kann. Hierdurch ist gewährleistet, dass der erste Femurkondylenschnitt 39 in der richtigen Höhe über dem Tibiaplateau ausgeführt wird.

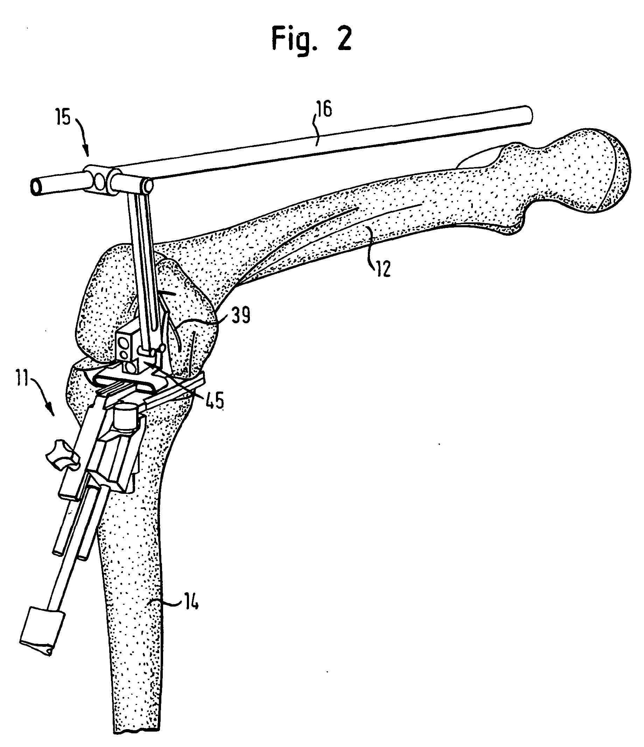

Entsprechend ist bei in Flexion befindlichem Knie gemäß Fig. 1 und 2 durch die Aufspreizeinrichtung 11 sichergestellt, dass Tibia 14 und Femur 12 ihre korrekte Relativlage zueinander einnehmen und somit die lösbar mit einem höhenverstellbar an der Aufspreizeinrichtung 11 angebrachten Funktionsaufsatz 13 koppelbare Schnitt- und Bohrlehre 45 bei korrekter Relativlage zwischen Femur 12 und Tibia 14 in einen an der Aufspreizeinrichtung 11 ablesbaren Sollabstand vom Tibiaplateau gebracht werden kann.

Die Aufspreizeinrichtung 11 umfasst ein Oberteil 19 mit einer plattenförmigen Spreizzunge 29 sowie ein Unterteil 17 mit einer ebenfalls plattenförmigen Spreizzunge 27. Mittels der beiden parallel zueinander verlaufenden Spreizzungen 27, 29 kann die Femurkondyle von dem Tibiaplateau weggedrückt werden, indem mittels eines als Stellschraube ausgebildeten Betätigungsorgans 21 das Oberteil 19 gegenüber dem Unterteil 17 angehoben wird.

Nachdem im Anschluss an das Aufspreizen des Knies die erfindungsgemäße Schnitt- und Bohrlehre 45 durch Verstellen des Funktionsaufsatzes 13 relativ zur Aufspreizeinrichtung 11 in die richtige Höhe über dem Tibiaplateau gebracht worden ist, wird der Funktionsaufsatz 13 mittels einer Feststelleinrichtung 49 an der Aufspreizeinrichtung 11 festgesetzt. Daraufhin wird die verschiebbar auf einem Kopfabschnitt des Funktionsaufsatzes 13 geführte Schnitt- und Bohrlehre 45 an die Femurkondyle herangeschoben.

Bevor die Schnitt- und Bohrlehre 45 an der Femurkondyle fixiert wird, wird sie mittels einer Ausrichthilfe 15 (vgl. Fig. 2) relativ zum Femur 12 ausgerichtet. Hierzu kann die Lehre 45 mittels der Ausrichthilfe 15 um eine im Wesentlichen senkrecht zum Tibiaplateau verlaufende Achse zusammen mit der Aufspreizeinrichtung 11 verdreht werden. Außerdem ist es möglich, die Lehre 45 zusammen mit der Aufspreizeinrichtung 11 parallel zum Tibiaplateau zu verschieben.

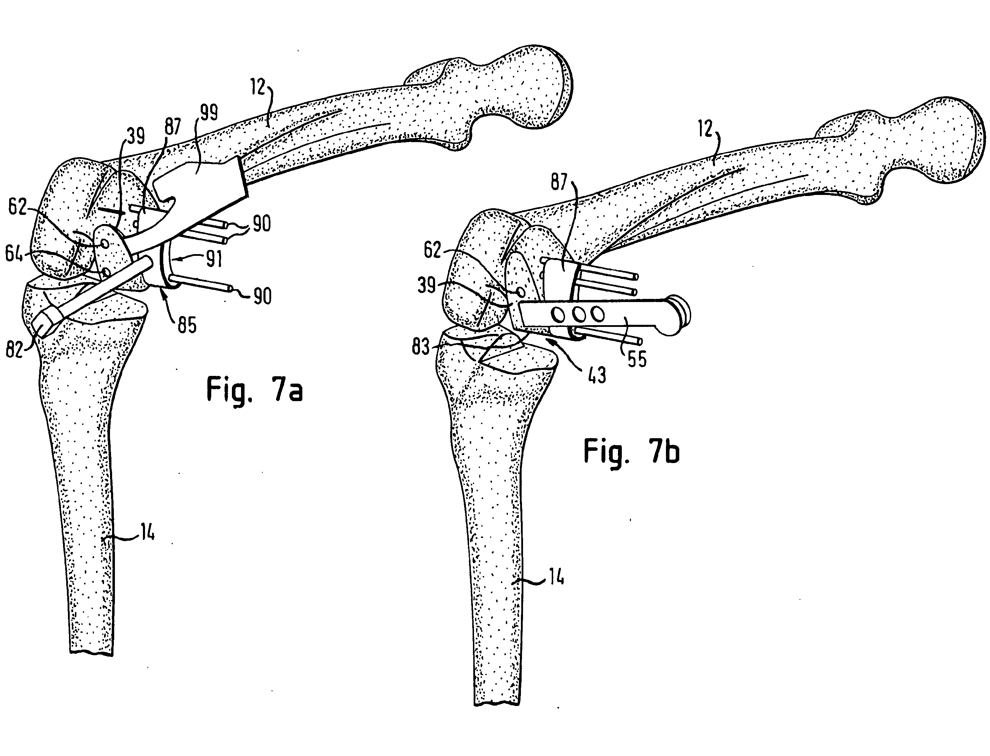

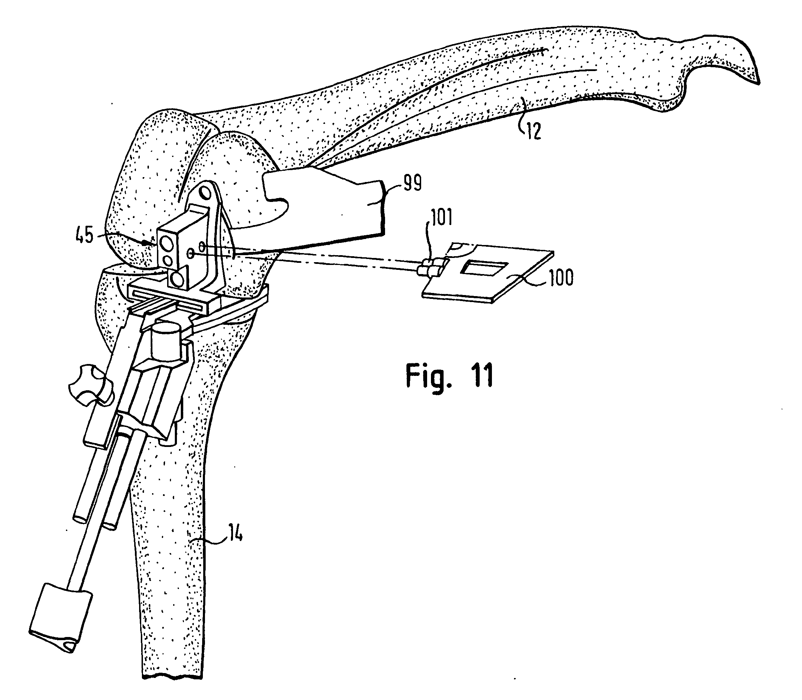

Das Ausrichten kann auch von Auge erfolgen, indem mit dem Bohrlehrenabschnitt 41 ein 90°-Winkel zu einem in den Schlitz 39 eingesteckten Anschlagblech (z.B. zu dem in Fig. 7a und Fig. 11 gezeigten Blech 99, das in Fig. 7a zu einem anderen Zweck verwendet wird) eingestellt wird. Eine weitere Ausrichtungsmöglichkeit gemäß Fig. 11 besteht darin, in die Kopplungsdurchgänge 73 des Bohrlehrenabschnitts 41 ein mit Stiften 101 versehenes, in der während der Benutzung dem Knie zugewandten Ecke ausgespartes 90° Winkelblech 100 einzustecken, welches eine Kontrolle des Spaltes zum Anschlagblech 99 auf Parallelität relativ einfach macht.

Die ein mehrteiliges Gestänge mit einer am Femur 12 ausrichtbaren Justierstange 16 umfassende Ausrichthilfe 15 ist seitlich in zwei im Bohrlehrenabschnitt 41 der Lehre 45 ausgebildete, gemeinsam einen Kopplungsabschnitt 23 der Lehre 45 bildende Kopplungsdurchgänge 73 steckbar.

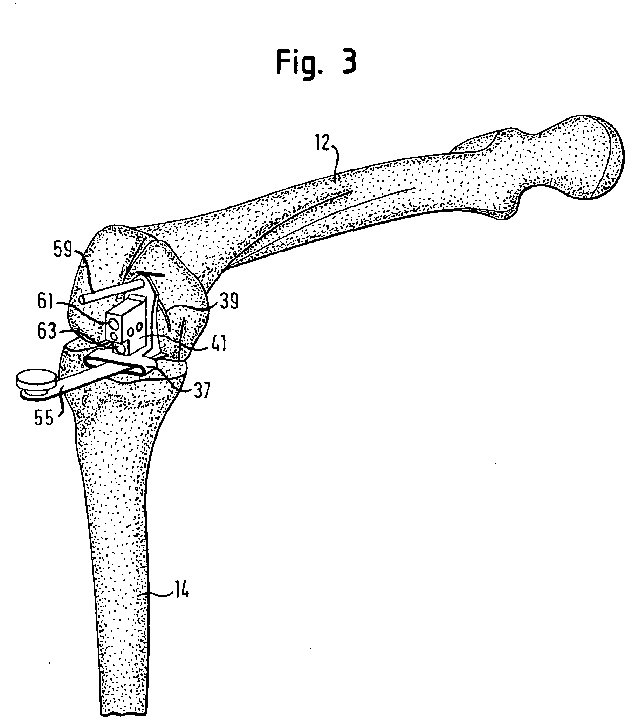

Anschließend wird die korrekt ausgerichtete Lehre 45 mittels eines durch einen Positionierdurchgang der Lehre 45 hindurchgeführten Positionierstiftes 59 (vgl. Fig. 3) an der Femurkondyle positioniert, woraufhin über in der Lehre 45 ausgebildete Fixierdurchgänge in der Femurkondyle Bohrungen für in Form von Knochenschrauben vorgesehene Fixierelemente 61, 63 hergestellt werden, mittels welcher die Lehre 45 an der Femurkondyle befestigt wird.

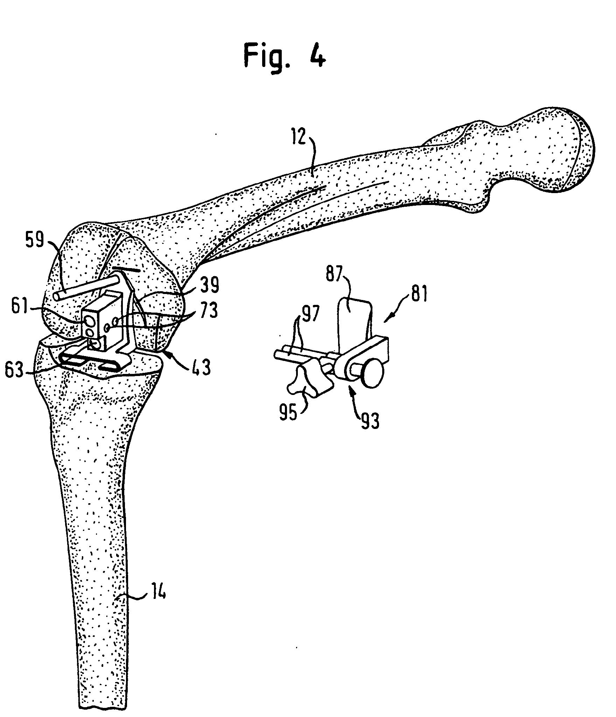

Dann wird die Aufspreizeinrichtung 11 abgenommen und mittels eines durch einen im Schnittlehrenabschnitt 37 ausgebildeten Schlitz 57 hindurchgeführten Schneidewerkzeugs in Form eines Sägeblatts 55 ein zweiter Kondylenschnitt 43 (vgl. Fig. 4) ausgeführt, der im Wesentlichen parallel zum Tibiaplateau und damit im Wesentlichen senkrecht zum ersten Kondylenschnitt 39 verläuft.

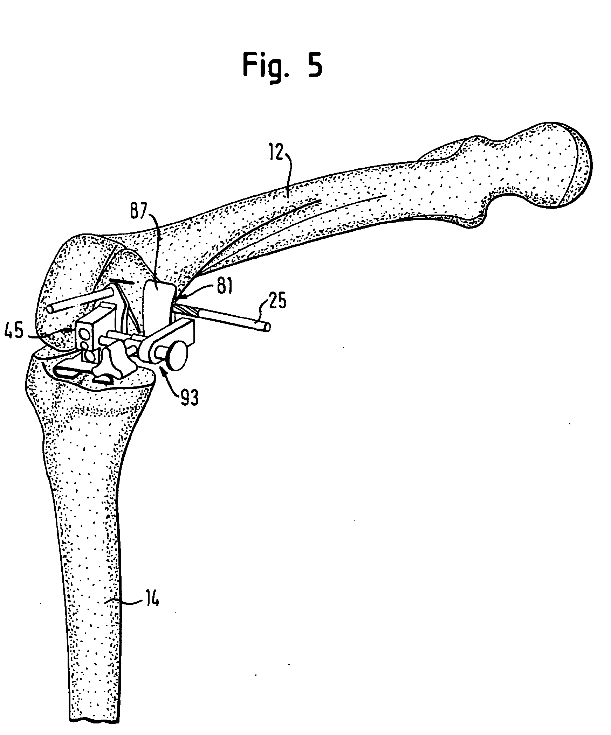

Als nächstes wird mit der nach wie vor an der Femurkondyle fixierten Lehre 45 über eine separate Kopplungseinrichtung 93 eine zusätzliche Schnittlehre 81 gekoppelt (vgl. Fig. 4 und 5), die dazu dient, einen entsprechend der der Femurkondyle zugewandten gekrümmten Seite der Lehre 45 verlaufenden weiteren Kondylenschnitt auszuführen.

Hierzu wird die zusätzliche Schnittlehre 81 mittels einer Spannvorrichtung 95 der Kopplungseinrichtung 93 an dieser eingespannt und anschließend über die Kopplungseinrichtung 93 in einer definierten Lage relativ zu der Lehre 45 mit dieser gekoppelt, indem Führungsstifte 97 der Kopplungseinrichtung 93 von der Seite her in die bereits erwähnten, auch zur Koppelung mit der Ausrichthilfe 15 (vgl. Fig. 2) vorgesehenen Kopplungsdurchgänge 73 gesteckt werden.

Die vorstehend erwähnte definierte Relativlage zeichnet sich dadurch aus, dass eine für ein Schneidewerkzeug vorgesehene Führungsfläche 87 der Schnittlehre 81 im über die Kopplungseinrichtung 93 mit der Lehre 45 gekoppelten Zustand derart bezüglich der gekrümmten Seite der Lehre 45 orientiert ist, dass die gekrümmte Seite der Lehre 45 und die Führungsfläche 87 lediglich translatorisch gegeneinander versetzt sind. Folglich kann über die Führungsfläche 87 der Verlauf der gekrümmten Seite der Lehre 45 auf die Femurkondyle übertragen werden, indem ein Schneidewerkzeug entlang der Führungsfläche 87 bewegt wird.

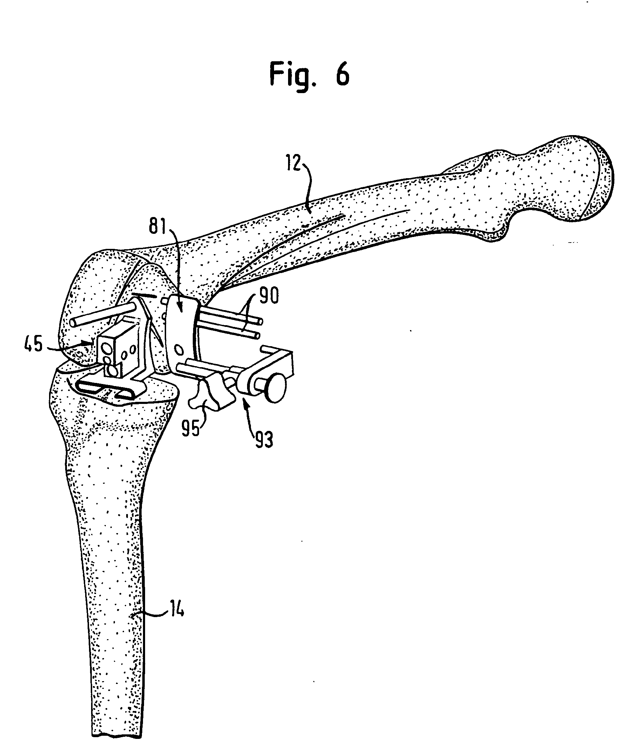

Bevor auf diese Weise der erwähnte weitere Kondylenschnitt ausgeführt wird, wird die zusätzliche Schnittlehre 81 ebenfalls an der Femurkondyle fixiert. Fig. 5 zeigt, wie hierzu über in der Schnittlehre 81 ausgebildete, im an die Lehre 45 gekoppelten Zustand senkrecht zu den Fixierdurchgängen der Lehre 45 verlaufende Fixierdurchgänge mittels eines Bohrers 25 die Femurkondyle vorgebohrt wird. Die Fig. 6, 7a und 7b zeigen die zusätzliche Schnittlehre 81 im mittels durch die Fixierdurchgänge hindurch in die Femurkondyle gesteckter Fixierstifte 90 an der Femurkondyle fixierten Zustand.

Die richtige Lage der zusätzlichen Schnittlehre 81 relativ zur Femurkondyle ist somit durch die ebenfalls korrekt positionierte und ausgerichtete Schnitt- und Bohrlehre 45 sichergestellt.

Die Kopplungseinrichtung 93 wird anschließend abgenommen, wie es in Fig. 6 angedeutet ist. Danach wird auch die kombinierte Schnitt- und Bohrlehre 45 von der Femurkondyle abgenommen.

Bevor mit Hilfe der Führungsfläche 87 der Schnittlehre 81 der weitere Kondylenschnitt ausgeführt wird, wird gemäß Fig. 7a mit Hilfe eines ebenen Anschlagbleches 99 und eines Einstellwerkzeugs 82 die Führungsfläche 87 relativ zur Femurkondyle verstellt, bis der Scheitelpunkt der konvexen Führungsfläche 87 in der durch den ersten Kondylenschnitt 39 definierten Ebene liegt. Hierzu wird das Anschlagblech 99 in den ersten Kondylenschnitt 39 eingeführt, wodurch die Schnittfläche des ersten Kondylenschnittes 39 verlängert und so ein Anschlag für die Führungsfläche 39 bereitgestellt wird.

Die Verstellbarkeit der Führungsfläche 87 ist dadurch gegeben, dass die Schnittlehre 81 mehrteilig ausgebildet ist und eine Kurvenscheibe 85, an der die Führungsfläche 87 ausgebildet ist, sowie einen Basisabschnitt 91 umfasst, der mit den Fixierdurchgängen für die Fixierstifte 90 versehen und über den die Schnittlehre 81 an der Femurkondyle fixiert ist.

Das Einstellwerkzeug 82 dient zur Betätigung einer die Kurvenscheibe 85 und den Basisabschnitt 91 miteinander verbindenden Stellschraube, deren mittels des Einstellwerkzeugs 82 bewirkte Drehbewegung eine Relativbewegung zwischen Kurvenscheibe 85 und Basisabschnitt 91 zur Folge hat, wodurch die Führungsfläche 87 vom Basisabschnitt 91 weg oder auf diesen zu bewegt wird.

Wenn der Scheitelpunkt der Führungsfläche 87 in der Ebene der ersten Schnittfläche 39 liegt, ist sichergestellt, dass das einzusetzende Femurimplantat sich in jeder Stellung des Kniegelenks im richtigen Abstand von dem einzusetzenden Tibiaimplantat befindet.

Fig. 7b zeigt, wie mittels eines entlang der Führungsfläche 87 geführten Schneidewerkzeugs in Form eines Sägeblatts 55 der gekrümmte weitere Kondylenschnitt ausgeführt wird, wodurch zwischen den beiden zuvor hergestellten ebenen Kondylenschnittflächen 39, 43 eine gekrümmte Kondylenschnittfläche 83 erzeugt wird, deren Verlauf der Krümmung der gekrümmten Seite der Schnitt- und Bohrlehre 45 und damit der entsprechenden Seite des einzusetzenden Femurimplantats entspricht.

In Fig. 7a und 7b sind die in der Femurkondyle in Verbindung mit der Fixierung der kombinierten Schnitt- und Bohrlehre 45 ausgebildeten Bohrungen 62, 64 zu erkennen, in die aufgrund der erfindungsgemäßen Ausbildung des Bohrlehrenabschnitts 41 der Lehre 45 entsprechend dem einzusetzenden Femurimplantat dessen Zapfen gesteckt werden können.

Nachdem der gekrümmte Kondylenschnitt 83 ausgeführt worden ist, wird die zusätzliche Schnittlehre 81 von der Femurkondyle abgenommen. Das gemäß der Operationsplanung ausgewählte Femurimplantat kann dann - gegebenenfalls nach Vornahme weiterer Vorbereitungsschritte, auf die hier nicht näher eingegangen werden soll - an der mittels der erfindungsgemäßen Vorrichtung vorbereiteten Femurkondyle fixiert werden.

Gemäß Fig. 8 ist die in verschiedenen, einem Satz von vorhandenen Femurimplantaten entsprechenden Größen vorgesehene kombinierte Schnitt- und Bohrlehre 45 im unteren Bereich als mit einem Schlitz 57 für ein Sägeblatt versehene Schnittlehre 37 und im oberen Bereich als mit zwei Fixierdurchgängen 69 versehene Bohrlehre 41 ausgebildet, durch die jeweils ein Bohrer zum Vorbohren des Knochens und anschließend ein Fixierelement 61, 63 (vgl. Fig. 3), insbesondere eine Knochenschraube zur Fixierung der Lehre 45 an der Femurkondyle, hindurchführbar sind.

Wie vorstehend erwähnt, entspricht die Lehre 45 hinsichtlich der Krümmung ihrer während der Operation der Femurkondyle zugewandten konkaven Seite 71 sowie hinsichtlich der Lage und der Orientierung der Fixierdurchgänge 69 bezüglich der gekrümmten Seite 71 einem Femurimplantat gleicher Größe. Insofern wird bei der Operation mittels der Lehre 45 das einzusetzende Femurimplantat simuliert.

An ihrem oberen Ende ist die Lehre 45 mit einem Positionierdurchgang 75 versehen, durch den der Positionierstift 59 (vgl. z.B. Fig. 3) hindurchgeführt wird, um die Lehre 45 an der Femurkondyle zu halten, während über die Fixierdurchgänge 69 in der Femurkondyle die Bohrungen 62, 64 für die Fixierschrauben 61, 63 bzw. für die Zapfen des einzusetzenden Femurimplantats ausgeführt werden.

Des Weiteren ist die Lehre 45 in dem Bereich zwischen den beiden Fixierdurchgängen 69 mit quer zu den Fixierdurchgängen 69 verlaufenden Kopplungsdurchgängen 73 versehen, die gemeinsam den Kopplungsabschnitt 23 des Bohrlehrenabschnitts 41 bilden.

Der Schnittlehrenabschnitt 37 der Lehre 45 ist gleichzeitig als Gleitsteckschuh ausgebildet, mit dem die Lehre 45 auf den Funktionsaufsatz 13 der Aufspreizeinrichtung 11 (vgl. Fig. 1) aufgesteckt und längs des Kopfabschnitts des Funktionsaufsatzes 13 verschoben werden kann.

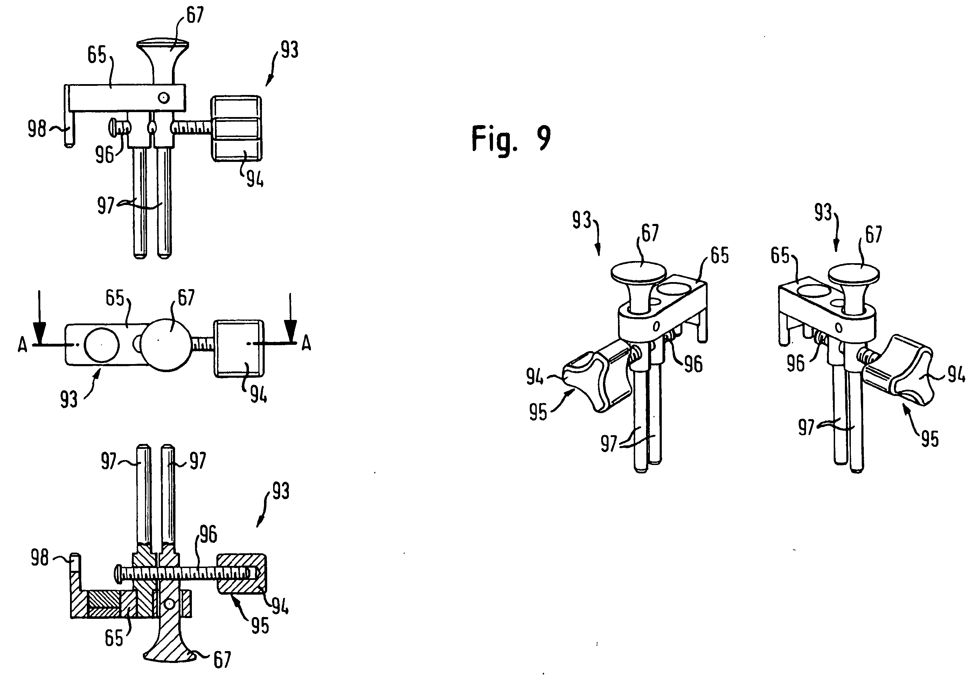

Die in Fig. 9 dargestellte separate Kopplungseinrichtung 93 umfasst zwei parallel verlaufende Führungsstifte 97, die fest mit einem sich senkrecht zu den Führungsstiften 97 erstreckenden Ausleger 65 verbunden sind, der an seinem einen Ende einen parallel zu den Führungsstiften 97 vorstehenden Anschlagabschnitt 98 aufweist. Der Abstand der beiden Führungsstifte 97 entspricht dem Abstand der in der erfindungsgemäßen Schnitt- und Bohrlehre 45 vgl. Fig. 8) ausgebildeten Kopplungsdurchgänge 73.

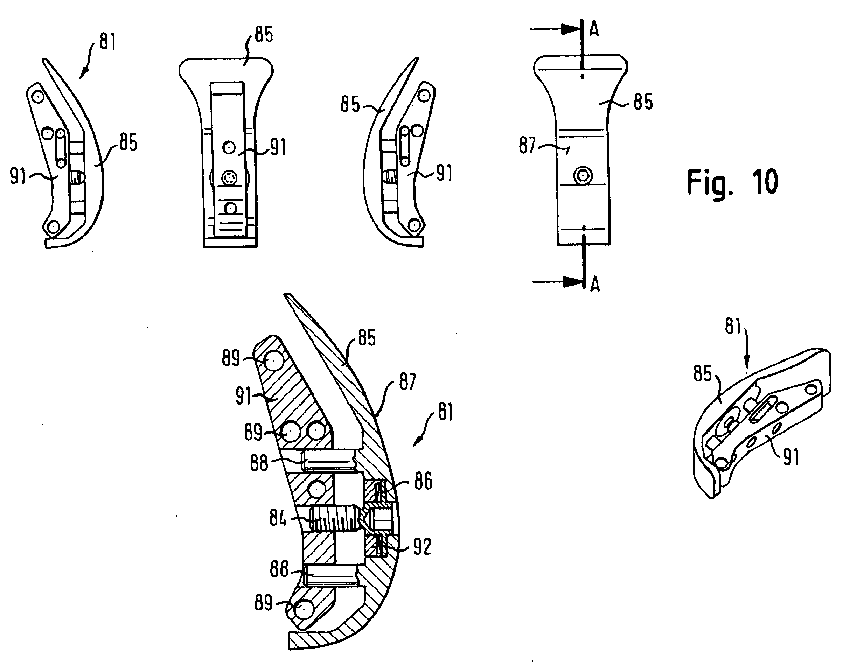

Zwischen dem Anschlagabschnitt 98 und dem freien Ende eines Klemmstempels 96, der einen Griffabschnitt 94 aufweist und Bestandteil einer Spannvorrichtung 95 der Kopplungseinrichtung 93 ist, kann die nachstehend näher in Verbindung mit Fig. 10 erläuterte zusätzliche Schnittlehre 81 in einer definierten Lage relativ zur Kopplungseinrichtung 93 eingespannt werden. Hierzu ist der Klemmstempel 96 als über den Griffabschnitt 94 drehbare Klemmschraube ausgebildet, die über ein Gewinde mit den Führungsstiften 97 zusammenwirkt.

Zur Handhabung der Kopplungseinrichtung 93 beim Verbinden mit und beim Lösen von der Schnitt- und Bohrlehre 45 kann der Operateur die Kopplungseinrichtung 93 mit der einen Hand an einem radial erweiterten Halteende 67 des einen Führungsstiftes 97 halten und mit der anderen Hand die Spannvorrichtung 95 über den Griffabschnitt 94 betätigen.

Gemäß Fig. 10 umfasst die entsprechend der Schnitt- und Bohrlehre 45 in verschiedenen, den vorhandenen Femurimplantatgrößen entsprechenden Größen vorgesehene zusätzliche Schnittlehre 81 einen drei Fixierdurchgänge 89 aufweisenden Basisabschnitt 91, relativ zu welchem eine mit der konvexen Führungsfläche 87 versehene Kurvenscheibe 85 verstellbar ist. Über in entsprechenden Durchgängen des Basisabschnitts 91 verschiebbar gelagerte Zapfen 88 ist die Kurvenscheibe 85 am Basisabschnitt 91 derart zwangsgeführt, dass lediglich eine geradlinige Relativbewegung zwischen Basisabschnitt 91 und Kurvenscheibe 85 möglich ist.

Das Verstellen der Kurvenscheibe 85 erfolgt mittels einer Stellschraube 84, die über einen mit einem Außengewinde versehenen Schaft mit einem Innengewinde zusammenwirkt, das in einem weiteren Durchgang des Basisabschnitts 91 ausgebildet ist. Der ringförmige Kopf der Stellschraube 84 ist zwischen der Rückseite der Kurvenscheibe 85 und einem als Federscheibe ausgebildeten Federelement 86 an der Kurvenscheibe 85 spielfrei in Verstellrichtung gehalten. Die Federscheibe 86 stützt sich an einem ringförmigen Abstützelement 92 ab, das fest mit der Kurvenscheibe 85 verbunden ist.

Mittels des in Fig. 7a dargestellten Einstellwerkzeugs wird die Stellschraube 84 in den mit dem Innengewinde versehenen Durchgang des Basisabschnitts 91 hinein oder aus diesem heraus geschraubt und auf diese Wiese die Kurvenscheibe 85 in der durch die Zapfen 88 bzw. die die Zapfen 88 aufnehmenden Durchgänge des Basisabschnitts 91 vorgegebenen Richtung relativ zum Basisabschnitt 91 verstellt.

Wie bereits erwähnt, entspricht der Verlauf der konvexen Führungsfläche 87 der Kurvenscheibe 85 der konkav gekrümmten Seite 71 der Schnitt- und Bohrlehre 45 (vgl. Fig. 8) und damit der entsprechenden Seite eines einzusetzenden Femurimplantats gleicher Größe.

Das freie Ende des Klemmstempels 96 der Kopplungseinrichtung 93 (vgl. Fig. 9) greift zum Einspannen der zusätzlichen Schnittlehre 81 an der in der Führungsfläche 87 ausgebildeten Öffnung an, über welche die Stellschraube 84 zugänglich ist. Hierzu ist das freie Ende des Klemmstempels 96 mit einer Abrundung versehen.

### Bezugszeichenliste

- 11: Aufspreizeinrichtung
- 12: Femur
- 13: Funktionsaufsatz
- 14: Tibia
- 15: Ausrichthilfe
- 16: Justierstange
- 17: Unterteil
- 19: Oberteil
- 21: Betätigungsorgan, Stellschraube
- 23: Kopplungsabschnitt
- 25: Bohrer
- 27: Spreizzunge des Unterteils
- 29: Spreizzunge des Oberteils
- 37: Schnittlehrenabschnitt
- 39: erster Kondylenschnitt, erste Schnittfläche
- 41: Bohrlehrenabschnitt
- 43: zweiter Kondylenschnitt, zweite Schnittfläche
- 45: kombinierte Schnitt- und Bohrlehre
- 49: Feststelleinrichtung
- 55: Schneidewerkzeug, Sägeblatt
- 57: Schlitz
- 59: Positionierstift
- 61: Fixierelement, Schraube
- 62: Bohrung
- 63: Fixierelement, Schraube
- 64: Bohrung
- 65: Ausleger
- 67: Halteende
- 69: Fixierdurchgang
- 71: gekrümmte Seite
- 73: Kopplungsdurchgang
- 75: Positionierdurchgang
- 81: zusätzliche Schnittlehre
- 82: Einstellwerkzeug
- 83: weiterer Kondylenschnitt, gekrümmte Schnittfläche
- 84: Stellschraube
- 85: Kurvenscheibe
- 86: Federelement, Federscheibe
- 87: Führungsfläche
- 88: Zapfen
- 89: Fixierdurchgang
- 90: Fixierstift
- 91: Basisabschnitt
- 92: Abstützelement
- 93: separate Kopplungseinrichtung
- 94: Griffabschnitt
- 95: Spannvorrichtung
- 96: Klemmstempel
- 97: Führungsstift
- 98: Anschlagabschnitt
- 99: Anschlagblech
- 100: Winkelblech
- 101: Stift

## Patentansprüche

1. Vorrichtung zur Vorbereitung einer Femurkondyle beim Einsetzen von monokondylären Knieimplantaten,
mit wenigstens einer kombinierten Schnitt- und Bohrlehre (45),
die bei in Flexion befindlichem Knie in einem von der Dicke eines einzusetzenden Tibiaimplantats abhängigen Sollabstand von einem Tibiaplateau an der Femurkondyle fixierbar ist,
deren der Femurkondyle zugewandte konkave Seite (71) mit einer Krümmung versehen ist, die dem Verlauf der den Femurkondyle zugewandten Seite eines einzusetzenden Femurimplantats aufweist,
die in einem Bohrlehrenabschnitt (41) wenigstens einen Fixierdurchgang (69) für einen Bohrer und für ein Fixierelement (61, 63) aufweist, wobei der Fixierdurchgang (69) bezüglich der gekrümmten Seite (71) entsprechend dem einzusetzenden Femurimplantat positioniert und orientiert und zum Vorbeneiden einer Bohrung für einen Zapfen des einzusetzenden Femurimplantats geeignet ist,
die in einem Schnittlehrenabschnitt (37) wenigstens einen Schlitz (57) für ein Schneidewerkzeug (55) aufweist, durch den eine Schnittebene für einen Kondylenschnitt (43) definiert ist, und
die einen Kopplungsabschnitt (23) für eine Ausrichthilfe (15) aufweist, mittels welcher die im Sollabstand vom Tibiaplateau befindliche Schnitt- und Bohrlehre (45) relativ zur Femurkondyle verstellbar ist.

2. Vorrichtung nach Anspruch 1,
**dadurch gekennzeichnet,**
**dass** als Kopplungsabschnitt (23) der Schnitt- und Bohrlehre (45) wenigstens ein im Bohrlehrenabschnitt (41) ausgebildeter Kopplungsdurchgang (73) vorgesehen ist, der im Wesentlichen senkrecht zu dem Fixierdurchgang (69) verläuft.

3. Vorrichtung nach Anspruch 1 oder 2,
**dadurch gekennzeichnet,**
**dass** die Schnitt- und Bohrlehre (45) mit einer Aufspreizeinrichtung (11) koppelbar ist, mittels welcher ein Sollabstand zwischen der Femurkondyle und einem gegenüberliegenden Tibiaplateau einstellbar ist.

4. Vorrichtung nach Anspruch 3,
**dadurch gekennzeichnet,**
**dass** der Schnittlehrenabschnitt (37) der Schnitt- und Bohrlehre (45) gleichzeitig als mit der Aufspreizeinrichtung (11) koppelbarer Gleitsteckschuh ausgebildet ist, über den die Schnitt- und Bohrlehre (45) an der Aufspreizeinrichtung (11) zwangsgeführt verstellbar ist.

5. Vorrichtung nach einem der vorhergehenden Ansprüche
**dadurch gekennzeichnet,**
**dass** die Schnitt- und Bohrlehre (45) zusätzlich zu dem Fixierdurchgang (69) mit einem im Bohrlehrenabschnitt (41) ausgebildeten Positionierdurchgang (75) versehen ist, über den die Schnitt- und Bohrlehre (45) vor der über den Fixierdurchgang (69) erfolgenden Fixierung an der Femurkondyle mittels eines Positionierstiftes (59) relativ zur Femurkondyle positionierbar ist.

6. Vorrichtung nach einem der vorhergehenden Ansprüche
**dadurch gekennzeichnet,**
**dass** die Schnitt- und Bohrlehre (45) mit einer zusätzlichen Schnittlehre (81) koppelbar ist, mit der bei in Flexion befindlichem Knie ein weiterer Kondylenschnitt (83) festlegbar ist, der gekrümmt zwischen zwei zuvor an der Femurkondyle hergestellten ebenen Schnittflächen (39, 43) verläuft, von denen die eine Schnittfläche (43) mittels des Schnittlehrenabschnitts (37) der kombinierten Schnitt- und Bohrlehre (45) und die andere Schnittfläche (39) bei in Extension befindlichem Knie ausgebildet wurde, wobei vorzugsweise die beiden ebenen Schnittflächen (39, 43) zumindest im Wesentlichen senkrecht zueinander verlaufen.

7. Vorrichtung nach Anspruch 6,
**dadurch gekennzeichnet,**
**dass** mittels der zusätzlichen Schnittlehre (81) der Verlauf der gekrümmten Seite (71) der kombinierten Schnitt- und Bohrlehre (45) zumindest bereichsweise auf der Femurkondyle abbildbar ist.

8. Vorrichtung nach Anspruch 6 oder 7,
**dadurch gekennzeichnet,**
**dass** die zusätzliche Schnittlehre (81) eine Kurvenscheibe (85) mit einer konvexen Führungsfläche (87) umfasst, entlang welcher ein Schneidewerkzeug (55) führbar ist und deren Verlauf der gekrümmten Seite (71) der kombinierten Schnitt- und Bohrlehre (45) entspricht.

9. Vorrichtung nach Anspruch 8,
**dadurch gekennzeichnet,**
**dass** die zusätzliche Schnittlehre (81) im mit der an der Femurkondyle fixierten Schnitt- und Bohrlehre (45) gekoppelten Zustand derart bezüglich der Schnitt- und Bohrlehre (45) orientiert ist, dass die Führungsfläche (87) der zusätzlichen Schnittlehre (81) und die gekrümmte Seite (71) der Schnitt- und Bohrlehre (45) ausschließlich translatorisch gegeneinander versetzt sind.

10. Vorrichtung nach einem der Ansprüche 6 bis 9,
**dadurch gekennzeichnet,**
**dass** die zusätzliche Schnittlehre (81) im mit der an der Femurkondyle fixierten Schnitt- und Bohrlehre (45) gekoppelten Zustand ebenfalls an der Femurkondyle fixierbar ist.

11. Vorrichtung nach einem der Ansprüche 6 bis 10,
**dadurch gekennzeichnet,**
**dass** die zusätzliche Schnittlehre (81) mit wenigstens einem und vorzugsweise einer Mehrzahl von Fixierdurchgängen (89) versehen ist, die sich im mit der kombinierten Schnitt- und Bohrlehre (45) gekoppelten Zustand im Wesentlichen senkrecht zu dem Fixierdurchgang (69) der kombinierten Schnitt- und Bohrlehre (45) erstrecken.

12. Vorrichtung nach einem der Ansprüche 8 bis 11,
**dadurch gekennzeichnet,**
**dass** die Führungsfläche (87) bei an der Femurkondyle fixierter zusätzlicher Schnittlehre (81) relativ zu der Femurkondyle verstellbar ist.

13. Vorrichtung nach einem der Ansprüche 8 bis 12,
**dadurch gekennzeichnet,**
**dass** die zusätzliche Schnittlehre (81) einen an der Femurkondyle fixierbaren Basisabschnitt (91) umfasst, an dem die Kurvenscheibe (85) bei an der Femurkondyle fixiertem Basisabschnitt (91) zur Ausrichtung der an der Kurvenscheibe (85) ausgebildeten Führungsfläche (87) mit einem Kondylenschnitt (39) verstellbar angebracht ist, der zuvor bei in Extension befindlichem Knie in einem von der Dicke eines einzusetzenden Tibiaimplantats abhängigen Sollabstand vom Tibiaplateau ausgeführt wurde.

14. Vorrichtung nach Anspruch 13,
**dadurch gekennzeichnet,**
**dass** die Kurvenscheibe (85) derart relativ zu dem an der Femurkondyle fixierten Basisabschnitt (91) verstellbar ist, dass der Scheitelpunkt der an der Kurvenscheibe (85) ausgebildeten Führungsfläche (87) in der durch den Kondylenschnitt (39) definierten Ebene gelegen ist.

15. Vorrichtung nach einem der Ansprüche 6 bis 14,
**dadurch gekennzeichnet,**
**dass** der für die Ausrichthilfe (15) vorgesehene Kopplungsabschnitt (23) der kombinierten Schnitt- und Bohrlehre (45) gleichzeitig zur Koppelung mit der zusätzlichen Schnittlehre (81) ausgebildet ist.

16. Vorrichtung nach einem der Ansprüche 6 bis 15,
**dadurch gekennzeichnet,**
**dass** zur Koppelung der zusätzlichen Schnittlehre (81) mit der kombinierten Schnitt- und Bohrlehre (45) eine separate Kopplungseinrichtung (93) vorgesehen ist, an der die zusätzliche Schnittlehre (81) fixierbar und mit der die zusätzliche Schnittlehre (81) im fixierten Zustand relativ zu der kombinierten Schnitt- und Bohrlehre (45) verstellbar, insbesondere linear verschiebbar, ist.

17. Vorrichtung nach Anspruch 16,
**dadurch gekennzeichnet,**
**dass** die Kopplungseinrichtung (93) eine Spannvorrichtung (95) umfasst, mittels welcher die zusätzliche Schnittlehre (81) an der Kopplungseinrichtung (93) fixierend einspannbar ist.

18. Vorrichtung nach Anspruch 16 oder 17,
**dadurch gekennzeichnet,**
**dass** die Kopplungseinrichtung (93) bei an der Femurkondyle fixierter kombinierter Schnitt- und Bohrlehre (45) und zusätzlicher Schnittlehre (81) abnehmbar ist.

## Claims

1. An apparatus for the preparation of a femoral condyle for the insertion of monocondylar knee implants,
comprising at least one combined cutting and drilling jig (45) which, when the knee is in flexion, can be fixed to the femoral condyle at a desired spacing from a tibia plateau dependent on the thickness of a tibia implant to be inserted,
the jig having a concave side (71) facing the femoral condyle and provided with a curvature which has the course of the side of a femur implant to be inserted facing the femoral condyle,
having at least one fixing passage (69) in a drilling section (41) of the jig for a drill and for a fixing element (61, 63), with the fixing passage (69) being positioned and oriented with respect to the curved side (71) in accordance with the femur implant to be inserted and being suitable for the preparation of a bore for a spigot of the femur implant to be inserted,
having at least one slot (57) in a cutting section (37) of the jig for a cutting tool (55) by which a cutting plane for a condylar cut (43) is defined, and
having a coupling section (23) for an alignment aid (15) by means of which the cutting and drilling jig (45) located at the desired spacing from the tibia plateau is adjustable relative to the femoral condyle.

2. An apparatus in accordance with claim 1, **characterized in that** at least one coupling passage (73) formed in the drilling section (41) of the jig and extending substantially perpendicular to the fixing passage (69) is provided as a coupling section (23) of the cutting and drilling jig (45).

3. An apparatus in accordance with claim 1 or claim 2, **characterized in that** the cutting and drilling jig (45) can be coupled to a spreading device (11) by means of which a desired spacing can be set between the femoral condyle and an oppositely disposed tibia plateau.

4. An apparatus in accordance with claim 3, **characterized in that** the cutting section (37) of the of the cutting and drilling jig (45) is simultaneously made as a slide attachment shoe which can be coupled to the spreading device (11) and via which the cutting and drilling jig (45) is adjustable in a compulsorily guided manner at the spreading device (11).

5. An apparatus in accordance with any one of the preceding claims **characterized in that** the cutting and drilling jig (45) is provided, in addition to the fixing passage (69), with a positioning passage (75) which is formed in the drilling section (41) of the jig and via which the cutting and drilling jig (45) is positionable relative to the femoral condyle by means of a positioning pin (59) before the fixing to the femoral condyle taking place via the fixing passage (69).

6. An apparatus in accordance with any one of the preceding claims **characterized in that** the cutting and drilling jig (45) can be coupled to an additional cutting jig (81) with which, when the knee is in flexion, a further condylar cut (83) can be fixed which extends in a curved manner between two planar cut surfaces (39, 43) which have previously been established at the femoral condyle and of which the one cut surface (43) was formed by means of the cutting section (37) of the combined cutting and drilling jig (45) and the other cut surface (39) was made when the knee was in extension, with the two planar cut surfaces (39, 43) preferably extending at least substantially perpendicular to one another.

7. An apparatus in accordance with claim 6, **characterized in that** the course of the curved side (71) of the combined cutting and drilling jig (45) is able to be mapped at least regionally on the femoral condyle by means of the additional cutting jig (81)

8. An apparatus in accordance with claim 6 or claim 7, **characterized in that** the additional cutting jig (81) includes a disk cam (85) with a convex guide surface (87) along which a cutting tool (55) is guidable and whose extent corresponds to the curved side (71) of the combined cutting and drilling jig (45).

9. An apparatus in accordance with claim 8, **characterized in that** the additional cutting jig (81) is oriented with respect to the cutting and drilling jig (45) in the state coupled to the cutting and drilling jig (45) fixed to the femoral condyle such that the guide surface (87) of the additional cutting jig (81) and the curved side (71) of the cutting and drilling jig (45) are only translatorily offset toward one another.

10. An apparatus in accordance with one of claims 6 to 9, **characterized in that** the additional cutting jig (81) is likewise fixable to the femoral condyle in the state coupled to the cutting and drilling jig (45) fixed to the femoral condyle.

11. An apparatus in accordance with one of claims 6 to 10, **characterized in that** the additional cutting jig (81) is provided with at least one fixing passage (89), and preferably a plurality of fixing passages, which extend substantially perpendicular to the fixing passage (69) of the combined cutting and drilling jig (45) in the state coupled to the combined cutting and drilling jig (45).

12. An apparatus in accordance with one of claims 8 to 11, **characterized in that** the guide surface (87) is adjustable relative to the femoral condyle with the additional cutting jig (81) fixed to the femoral condyle.

13. An apparatus in accordance with one of claims 8 to 12, **characterized in that** the additional cutting jig (81) includes a base section (91) which can be fixed to the femoral condyle and to which the disk cam (85) is adjustably attached with the base section (91) fixed to the femoral condyle for the alignment of the guide surface (87) formed at the cam disk (85) with a condylar cut (39) which was previously carried out, when the knee was in extension, at a desired spacing from the tibia plateau dependent on the thickness of a tibia implant to be inserted.

14. An apparatus in accordance with claim 13, **characterized in that** the disk cam (85) is adjustable relative to the base section (91) fixed to the femoral condyle such that the vertex of the guide surface (87) formed at the disk cam (85) is disposed in the plane defined by the condylar cut (39).

15. An apparatus in accordance with one of claims 6 to 14, **characterized in that** the coupling section (23) of the combined cutting and drilling jig (45) provided for the alignment aid (15) is simultaneously made for coupling to the additional cutting jig (81).

16. An apparatus in accordance with one of claims 6 to 15, **characterized in that** a separate coupling device (93) is provided for the coupling of the additional cutting jig (81) to the combined cutting and drilling jig (45) at which the additional cutting jig (81) can be fixed and with which the additional cutting jig (81) is adjustable, in particular linearly displaceable, relative to the combined cutting and drilling jig (45) in the fixed state.

17. An apparatus in accordance with claim 16, **characterized in that** the coupling device (93) includes a clamping device (95) by means of which the additional cutting jig (81) is fixingly clampable to the coupling device (93).

18. An apparatus in accordance with claim 16 or claim 17, **characterized in that** the coupling device (93) is removable with the combined cutting and drilling jig (45) and the additional cutting jig (81) fixed to the femoral condyle.

## Revendications

1. Dispositif destiné à la préparation d'un condyle fémoral lors de la mise en place d'implants monocondyliens de genoux,
comportant au moins un gabarit de coupe et de perçage combiné (45) qui, à l'état fléchi du genou, peut être fixé sur le condyle fémoral à un écartement prescrit d'un plateau de tibia dépendant de l'épaisseur d'un implant tibial à mettre en place, dont la face concave (71) orientée vers le condyle fémoral est pourvue d'une incurvation dont l'extension correspond à la face orientée vers le condyle fémoral d'un implant fémoral à mettre en place,
lequel comporte dans un tronçon de gabarit de perçage (41) au moins un passage de fixation (69) pour un foret et pour un élément de fixation (61, 63), le passage de fixation (69) pouvant être positionné et orienté par rapport à la face incurvée (71) en fonction de l'implant fémoral à mettre en place et qui est approprié pour la préparation d'un perçage pour une cheville de l'implant fémoral à mettre en place,
lequel comporte dans un tronçon de gabarit de coupe (37) au moins une fente (57) pour un outil de coupe (55) qui définit un plan de coupe pour une coupe condylienne (43), et
lequel comporte un tronçon de couplage (23) pour une aide à l'orientation (15), au moyen de laquelle le gabarit de coupe et de perçage (45) se trouvant à un écartement prescrit du plateau de tibia peut être déplacé par rapport au condyle fémoral.

2. Dispositif selon la revendication 1,
**caractérisé en ce que**,
en tant que tronçon de couplage (23) du gabarit de coupe et de perçage (45), il est prévu au moins un passage de couplage (73) agencé dans le tronçon de gabarit de perçage (41), qui s'étend pour l'essentiel perpendiculairement au passage de fixation (69).

3. Dispositif selon la revendication 1 ou 2,
**caractérisé en ce que**
le gabarit de coupe et de perçage (45) peut être couplé à un dispositif écarteur (11), au moyen duquel un écartement prescrit entre le condyle fémoral et un plateau de tibia situé en face est réglable.

4. Dispositif selon la revendication 3,
**caractérisé en ce que**
le tronçon de gabarit de coupe (37) du gabarit de coupe et de perçage (45) est simultanément agencé sous la forme d'un patin d'emboîtement pouvant être couplé au dispositif écarteur (11), par l'intermédiaire duquel le gabarit de coupe et de perçage (45) peut être déplacé par guidage forcé au niveau du dispositif écarteur (11).

5. Dispositif selon l'une des revendications précédentes,
**caractérisé en ce que**,
en plus du passage de fixation (69), le gabarit de coupe et de perçage (45) est doté d'un passage de positionnement (75) agencé dans le tronçon de gabarit de perçage (41), par l'intermédiaire duquel le gabarit de coupe et de perçage (45) peut être positionné par rapport au condyle fémoral au moyen d'une broche de positionnement (59) avant la fixation au condyle fémoral effectuée par l'intermédiaire du passage de fixation (69).

6. Dispositif selon l'une des revendications précédentes,
**caractérisé en ce que**
le gabarit de coupe et de perçage (45) peut être couplé à un gabarit de coupe supplémentaire (81), avec lequel une autre coupe condylienne (83) peut être effectuée à l'état fléchi du genou, laquelle s'étend de façon incurvée entre deux surfaces de coupe (39, 43) planes du condyle fémoral réalisées auparavant, dont une surface de coupe (43) a été réalisée au moyen du tronçon de gabarit de coupe (37) du gabarit de coupe et de perçage combiné (45), et l'autre surface de coupe (39) à l'état en extension du genou, les deux surfaces de coupe (39, 43) planes étant de préférence au moins sensiblement perpendiculaires l'une par rapport à l'autre.

7. Dispositif selon la revendication 6,
**caractérisé en ce que**,
au moyen du gabarit de coupe supplémentaire (81), l'extension de la face incurvée (71) du gabarit de coupe et de perçage combiné (45) peut être reproduite sur le condyle fémoral, au moins par zones.

8. Dispositif selon la revendication 6 ou 7,
**caractérisé en ce que**
le gabarit de coupe supplémentaire (81) comporte une came (85) avec une surface de guidage convexe (87), le long de laquelle peut être guidé un outil de coupe (55), et dont l'extension correspond à la face incurvée (71) du gabarit de coupe et de perçage combiné (45).

9. Dispositif selon la revendication 8,
**caractérisé en ce que**,
à l'état couplé au gabarit de coupe et de perçage (45) fixé au condyle fémoral, le gabarit de coupe supplémentaire (81) est orienté par rapport au gabarit de coupe et de perçage (45) de telle sorte que la surface de guidage (87) du gabarit de coupe supplémentaire (81) et la face incurvée (71) du gabarit de coupe et de perçage (45) soient exclusivement décalées par translation l'une par rapport à l'autre.

10. Dispositif selon l'une des revendications 6 à 9,
**caractérisé en ce que**,
à l'état couplé au gabarit de coupe et de perçage (45) fixé au condyle fémoral, le gabarit de coupe supplémentaire (81) peut également être fixé au condyle fémoral.

11. Dispositif selon l'une des revendications 6 à 10,
**caractérisé en ce que**
le gabarit de coupe supplémentaire (81) est muni d'au moins un, et de préférence d'une pluralité de passages de fixation (89) qui, à l'état couplé au gabarit de coupe et de perçage combiné (45), s'étendent pour l'essentiel perpendiculairement au passage de fixation (69) du gabarit de coupe et de perçage combiné (45).

12. Dispositif selon l'une des revendications 8 à 11,
**caractérisé en ce que**
la surface de guidage (87) peut être déplacée par rapport au condyle fémoral lorsque le gabarit de coupe supplémentaire (81) est fixé au condyle fémoral.

13. Dispositif selon l'une des revendications 8 à 12,
**caractérisé en ce que**
le gabarit de coupe supplémentaire (81) comporte un tronçon de base (91) pouvant être fixé au condyle fémoral, sur lequel tronçon de base (91) fixé au condyle fémoral, la came (85) est disposée de façon à pouvoir être déplacée pour l'alignement de la surface de guidage (87) formée sur la came (85), avec une coupe condylienne (39) ayant été réalisée auparavant à l'état en extension du genou à un écartement prescrit du plateau de tibia dépendant de l'épaisseur d'un implant tibial à mettre en place.

14. Dispositif selon la revendication 13,
**caractérisé en ce que**
la came (85) peut être déplacée par rapport au tronçon de base (91) fixé au condyle fémoral de telle sorte que le point sommital de la surface de guidage (87) formée sur la came (85) soit situé dans le plan défini par la coupe condylienne (39).

15. Dispositif selon l'une des revendications 6 à 14,
**caractérisé en ce que**
le tronçon de couplage (23) du gabarit de coupe et de perçage combiné (45), prévu pour l'aide à l'orientation (15), est simultanément agencé pour le couplage au gabarit de coupe supplémentaire (81).

16. Dispositif selon l'une des revendications 6 à 15,
**caractérisé en ce que**,
pour le couplage du gabarit de coupe supplémentaire (81) au gabarit de coupe et de perçage combiné (45), il est prévu un dispositif de couplage (93) séparé, sur lequel peut être fixé le gabarit de coupe supplémentaire (81), et avec lequel le gabarit de coupe supplémentaire (81) à l'état fixé peut être déplacé par rapport au gabarit de coupe et de perçage combiné (45), notamment de façon linéaire.

17. Dispositif selon la revendication 16,
**caractérisé en ce que**
le dispositif de couplage (93) comporte un système de tension (95), au moyen duquel le gabarit de coupe supplémentaire (81) peut être fixé par serrage au dispositif de couplage (93).

18. Dispositif selon la revendication 16 ou 17,
**caractérisé en ce que**,
à l'état fixé au condyle fémoral du gabarit de coupe et de perçage combiné (45) et du gabarit de coupe supplémentaire (81), le dispositif de couplage (93) peut être retiré.
